# EUROPEAN PATENT APPLICATION

(11) **EP 2 801 322 A1**
(43) Date of publication of application: **12.11.2014**
(21) Application number: 14167574.4
(22) Date of filing: 08.05.2014
(51) Int. Cl.: A61B 7/04

(54) **Stethoscope with digital support**

(30) Priority: 09.05.2013 IT MI20130760
(71) Applicant: DOT S.r.l., 20025 Legnano (Milano) (IT)
(72) Inventor: Torpia, Roberto, 20025 Legnano (Milano) (IT)
(74) Representative: Petruzziello, Aldo

(57) **Abstract**

A stethoscope with digital support (10) suitable for auscultation of sound signals emitted by the organs of the human or animal body for diagnostic purposes, comprising a head or pavilion (12) suitable for coming into contact with the part to be auscultated and provided below with a recess or chamber (26), tendentially concave, suitable for conveying the sound signals in the direction of a harmonic box (37) formed in a through duct (30) developed in the pavilion (12) and wherein a microphone (34) is placed, suitable for acquiring and transmitting said sound signals to a computer, tablet or smartphone for the processing, representation and management of the signals, the head or pavilion (12) integrating mechanical means suitable for cutting or filtering unwanted audio frequencies and ambient noises.

## Description

The object of the present invention is a stethoscope with digital support.

More particularly the present invention relates to a stethoscope suitable for allowing the auscultation of the internal organs in general with digital technology.

As is known, the stethoscope is a device designed with the purpose of detecting the sounds emitted by the organs of the human or animal body (for example the heartbeat or the sounds emitted by joints, arteries, etc.) for diagnostic purposes. It improves and facilitates the auscultation of these sounds which, originally, were detected by the doctor simply by resting an ear directly on the body of the patient with evident difficulties of listening and consequent correct interpretation.

The stethoscope can be of the acoustic type, whose functioning takes place by means of transmission of the sounds from the chest or from other parts of the body of the patient to the ears of the listener (generally the doctor) via channels containing air. The end part or head of the instrument is positioned on the point to be ausculted of the patient and can have a disk or bell shape respectively suitable for allowing the transmission of sound waves at a higher frequency and at low frequency.

However the acoustic stethoscope has a major disadvantage linked to the low volume of the sound transmitted which entails a complexity of listening and a consequent difficulty in the performance of an accurate diagnosis.

In order to overcome this disadvantage an electronic/digital stethoscope has been developed in which the end part is provided with a microphone suitable for acquiring the sound waves which are then transformed into electrical impulses which can be amplified for easier listening by the doctor.

This stethoscope is typically provided with an appropriate filter of the ANR (ambient noise reduction) type, placed inside the head or end part together with all the electronic components and suitable for eliminating the external sound interferences in order to allow an acquisition of signals free from noises or other forms of disturbance which can "foul" the same signal.

However this type of stethoscope also has some disadvantages linked to the fact that the electronic components are all placed inside the head or end part of the instrument with a consequent increase in the sizes and/or overall dimensions and of the weight of the same.

A further disadvantage of the electronic/digital stethoscope is represented by the need to have one or more batteries, placed inside the head or end part and suitable for guaranteeing the power supply of the electronic components of the same instrument.

A further disadvantage is represented by the fact that the use of the electronic components integrated directly in the head or end part of the stethoscope makes the instrument very complex constructionally and, at the same time, costly, having to use electronic components with particularly small sizes.

A further known device suitable for detecting cardiac sounds is described in US2007/0032728 which defines a sensor suitable for allowing the acquisition of cardiac noises and tones for producing a phonocardiogram and which comprises a sensor of the piezoelectric transducer type placed inside a covering element operating as mechanical filter and provided with a regulation element suitable for regulating the position of said sensor with reference to the surface of the chest of the patient whereto said sensor is applied, with support means having the function of allowing the coupling of the covering element with respect to the surface of the chest of the patient and with means suitable for securing the covering element with respect to the chest of the patient, with said means typically defined by a strip of adhesive material wound on the chest of the patient so as to avoid movements of the sensor with respect to the positions of contact at which the auscultation of the cardiac noises and tones is performed.

However this sensor performs the auscultation of the cardiac sounds by means of an action of direct pressure on the chest of the patient and regulated by means of regulation of the screw or spring type and for whose functioning the securing to the chest of the patient by means of adhesive strips is necessary.

The object of the present invention is that of avoiding the disadvantages mentioned above.

More particularly the object of the present invention is that of providing a stethoscope with digital support with reduced weight and overall dimensions.

A further object of the present invention is that of providing a stethoscope which does not require the use of an electronic filter of the ANR type.

A further object of the present invention is that of providing a stethoscope with digital support which allows an easy and convenient acquisition of the sound signals emitted by the internal organs of an individual (or of an animal) and a subsequent processing by means of software of the same signals for an optimal and more accurate interpretation and an optional storage of said signals for comparison analysis, diagnosis, monitoring and similar medical activities.

A further object of the present invention is to make available to users a device of the electronic/digital stethoscope type suitable for ensuring a high level of resistance and reliability in time and such, moreover, as to be easily and economically produced.

These and other objects are achieved by the invention which has the features as claimed in the independent claim 1.

According to the invention a stethoscope with digital support is provided comprising a head or pavilion suitable for coming into contact with the part to be auscultated and provided below with a recess or chamber, tendentially concave, suitable for conveying the sound signals in the direction of a harmonic box formed in a through duct developed in the pavilion and wherein a microphone is placed, suitable for acquiring and transmitting said sound signals to a computer, tablet or smartphone for the processing, representation and management of the signals, the head or pavilion integrating, likewise, mechanical means suitable for cutting or filtering unwanted audio frequencies and ambient noises.

Advantageous embodiments of the invention are disclosed by the dependent claims.

The constructional and functional features of the stethoscope with digital support of the present invention will be made clearer by the following detailed description, in which reference is made to the accompanying drawings which represent a preferred and non-limiting embodiment thereof and in which:
Figure 1 represents schematically a side view of the stethoscope with digital support of the invention including its constituent components and accessories and partially sectioned;
Figure 2 represents schematically a further side view in partial section of the stethoscope of the invention;

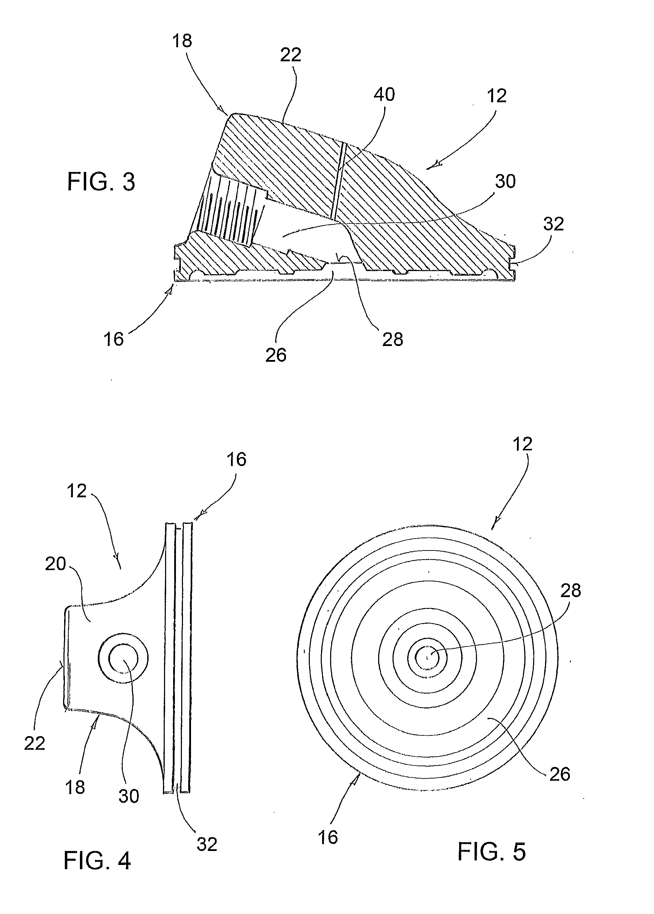

Figure 3 represents schematically a longitudinal section view of the head or end part of the stethoscope of the invention;
Figures 4 and 5 represent schematically the head of the stethoscope, respectively, according to a rear view and a view from below.

Referring to the aforesaid drawings the stethoscope with digital support of the present invention, denoted overall by 10, comprises a head or pavilion 12 suitable for being placed in contact with the body of the patient for the reception of the sounds and whereto a cable 14 is attached for the transmission of the sound waves detected by said head or pavilion 12 according to the methods detailed here below.

The pavilion 12 is typically made in pressure-cast composite alloy of aluminium or in another known and suitable material and comprises a body defined by a base portion 16 with tendentially circular development from whose upper face, opposite the one suitable for being placed in contact with the body of the patient, develops a body or portion 18 comprising a lateral surface 20 and an upper surface 22 suitable for allowing the gripping by two or more fingers of the hands of the doctor or of the user of the instrument.

The base portion 16 of the head or pavilion 12 develops, from the side opposite to that of the body or portion 18, a recess or chamber 26, tendentially concave, suitable for allowing the collection and the conveying of the sounds in the direction of a hole 28 formed centrally and at the top or maximum point of the concave profile of the aforementioned chamber. Said hole 28 places in communication the chamber 26 with a through duct 30 developed inside the body or portion 18 of the pavilion 12 from the lateral surface 20.

Along the outer lateral edge of the base portion 16 develops an annular recess 32 suitable for allowing the arrangement and securing of a gasket (not shown in the drawings) supporting a diaphragm (also not shown in the drawings) for closure of the base of said base portion.

Inside the through duct 30 is housed a microphone 34 of the condenser type and, in particular, an electret microphone which does not need an external power supply for the polarisation.

A silicone diaphragm 35 of the single-component type is placed in the through duct 30 upstream of the microphone 34 and a harmonic box or chamber 37 is formed between the hole 28 and the microphone 34. This silicone diaphragm is injected in said through duct 30 after the insertion of the microphone so as to render the same totally integrated with the structure of the head or pavilion and allow the decrease in the noises coming from the outside.

The head or pavilion 12 has a channel 40, formed starting from the upper surface 22 of the body or portion 18 and suitable for opening and placing in communication with the exterior the harmonic box or chamber 37 (otherwise closed) defined by the assembly of the microphone 34 and silicone diaphragm 35 inside the through duct 30.

Said channel 40 allows the regulation of the pressure between the chamber 37 and the entrance and defines a bandpass filter for the unwanted audio frequencies. The diameter of said channel is chosen as a function of the cutting of the unwanted frequencies and of the decrease of the redundancies between the fluctuating diaphragm for closure of the base of the base portion 16 of the pavilion 12 and the microphone 34.

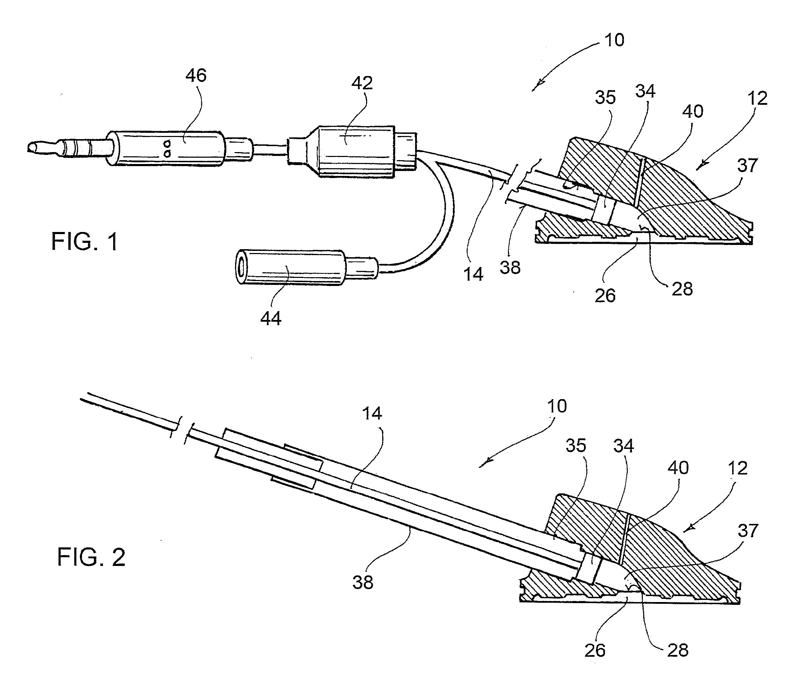

The microphone 34 is connected to the cable 14 for the sending of the signals acquired by the microphone in the direction of the device (not shown in the drawings) whose functions will be described here below. The cable 14 is partially screened by means of a sheath 38 (schematised in Figure 2) attached to the head or pavilion 12 at the through duct 30.

Referring to the preferred embodiment as shown in the drawings, the cable 14 integrates a baffle 42 for addressing the sound signals towards dedicated lines defined by a male connector or jack 46 for the transmission of the signals acquired in the direction of processing and interface means such as, for example, a computer or tablet or telephone of the smartphone type or by an additional female connector or jack 44 constituting an output line for example for connecting headphones (not shown in the drawing).

The stethoscope with digital support, described in detail above with reference to the structural features, is associated with a software application or program having the function of collecting, processing and presenting graphically the signals acquired by means of the same stethoscope. Said software application is installed in the remote device of the computer, tablet, smartphone type or the like and allows the activation, power supply and functioning of the stethoscope.

This software application processes the signals acquired by means of the pavilion 12 of the stethoscope and transmitted via the cable 14 allowing, in this way, the user to select the optimal listening frequencies and the correlated digital filters for different types of signals such as, for example, the cardiac or cardiopulmonary or pulmonary signal.

The software application, once the signals have been acquired and processed as defined above, produces a graph of the specific signal or sonogram (for example a "cardiac sonogram" in the case of a cardiac signal) and represents it on the screen of the smartphone or tablet or another suitable device.

The same application moreover allows the storage of the signals relating to the single users and their saving in chronological order in an archive located in the physical memory of the smartphone or of the tablet or the like or in a central and remote server. In this way it is possible to analyse the sound footprint, perform the comparison of the same with what is present in the archive so as to facilitate the diagnosis and, at the same time, make it more correct.

The functioning of the stethoscope with digital support of the present invention, described above with reference to its constituent components, is explained here below.

The user, defined for example by the doctor or by specialist staff or by the same patient/user, connects the stethoscope to the device of acquisition/processing and interface (table, smartphone, computer) by means of the male connector or jack 46 and optionally to headphones by means of the additional female connector or jack 44, activates the software application or program present on the acquisition/processing device and places the head or pavilion 12 of the stethoscope in contact with the part to be ausculted. The body signal acquired by means of the microphone 34 is sent to the acquisition/processing device via the cable 14 for the processing by the software application according to what is detailed above.

As can be seen from the above the advantages that the stethoscope of the invention achieves are clear.

The stethoscope with digital support of the present invention allows advantageously the performance of the auscultation of the sounds of the (human or animal) organism simply and easily also by an inexpert user.

A further advantage of the present invention is represented by the fact that the stethoscope with digital support is simple and economical to make with reference to the electronic components placed inside the head or pavilion and constituted by the microphone alone. In fact the remaining electronic components, suitable for the processing and management of the signals acquired via the head or pavilion, are made in digital form by means of the software application.

Further advantageous is the fact that the microphone, completely integrated in the structure of the pavilion by means of the silicone diaphragm, allows a considerable reduction in the noises coming from the outside, avoiding the use of electronic filters of the ANR type for the reduction of the ambient noises and, moreover, the presence of the channel 40 suitable for opening the harmonic box or chamber 37 allows the cutting of the unwanted audio frequencies, avoiding the use of bandpass filters placed upstream of the same microphone and inside the head or pavilion.

Further advantageous is the fact that the stethoscope with digital support of the present invention allows a use also in the sphere of remote medicine or telemedicine. This is because the signals acquired and stored via the software application can be sent to the doctor (by email, Bluetooth or with other known communication protocols) who can in this way perform possible analyses and process diagnoses even without the physical presence of the patient/user. This is because the software application also allows the storage of the sound traces and their reproduction.

Although the invention has been described above with particular reference to one of its embodiments given solely by way of a non-limiting example, numerous changes and variations will appear clear to a person skilled in the art in light of the description given above. The present invention therefore intends to embrace all the changes and variations which come within the scope and spirit of the following claims.

## Claims

1. A stethoscope with digital support (10) suitable for auscultation of sound signals emitted by the organs of the human or animal body, comprising a head or pavilion (12) suitable for coming into contact with the part to be auscultated and provided below with a recess or chamber (26) tendentially concave, closed below by means of a diaphragm and suitable for conveying the sound signals in the direction of a harmonic box (37) formed in a through duct (30) developed in the pavilion (12) and wherein a microphone (34) is placed, suitable for acquiring and transmitting said sound signals to a computer, tablet or smartphone for the processing, representation and management of the signals, said stethoscope **characterised in that** comprises a channel (40) formed starting from an outer surface of said head or pavilion (12) and suitable for placing in communication with the exterior the harmonic box or chamber (37) so as to regulate the pressure in said harmonic box or chamber and perform cutting and filtering of the unwanted audio frequencies and of ambient noises.

2. The stethoscope according to claim 1, **characterised in that** the microphone (34) is integrated in the structure of said pavilion (12) by means of a silicone diaphragm (35) placed in the through duct (30) upstream of the microphone itself and injected in said through duct after the insertion of the microphone.

3. The stethoscope according to any one of the preceding claims, **characterised in that** it comprises a cable (14) attached to the head or pavilion (12) at the through duct (30), partially shielded by means of a sheath (38) attached to the head or pavilion (12) and integrating a baffle (42) for addressing the sound signals acquired by means of the head or pavilion towards dedicated lines defined by a male connector or jack (46) for the transmission of the signals acquired in the direction of the remote acquisition device and by an additional female connector or jack (44) constituting an output line for connecting headphones.

4. The stethoscope according to claim 1, **characterised in that** the microphone (34) is an electret microphone.

5. The stethoscope according to any one of claims 1 to 4, **characterised in that** the head or pavilion (12) comprises a base portion (16) with circular development from whose upper face, opposite to the one suitable for being placed in contact with the body of the patient/animal, develops a body or portion (18) comprising a lateral surface (20) and an upper surface (22) suitable for allowing the gripping of the head or pavilion itself, the base portion (16) which comprises, on the side opposite to that of the body or portion (18), the recess or chamber (26) tendentially concave and provided with a hole (28) formed centrally to said chamber (26) at the maximum point of its concave profile and suitable for placing the chamber (26) in communication with the through duct (30) developed inside the body or portion (18) starting from the lateral surface (20), the head or pavilion (12) which likewise comprises a channel (40) formed starting from the upper surface (22) of the body or portion (18) and suitable for opening and placing in communication with the exterior the harmonic box or chamber (37).

6. The stethoscope according to any one of the preceding claims, **characterised in that** the base portion (16) of the head or pavilion (12) develops, along the outer side edge, an annular recess (32) suitable for allowing the placing and securing of a gasket supporting a diaphragm for closure of the base of said base portion.

7. Method of use of a stethoscope with digital support according to one or more of the preceding claims, **characterised in that** it comprises the following steps:
- connection of the stethoscope (10) to a computer, tablet or smartphone, by means of the connector (46) of the cable (14),
- optional connection of headphones to the stethoscope by means of the connector (44) of the cable (14);
- activation of a software application installed in the computer, tablet, smartphone suitable for processing the sound signals acquired through the pavilion or head (12) of the stethoscope, giving a visual/sound representation thereof and storing and/or transmitting them to a remote central server.
- acquisition of the sound signals emitted by the organ of the human/animal body via the microphone (34) of the head or pavilion (12) in contact with the portion of the body involved and sending to the software application;
- selection of the optimum listening frequencies and of the related digital filters for the different types of sound signals acquired of the cardiac or cardiopulmonary, pulmonary signal type;
- generation and representation of a graph or sonogram of the acquired and processed signal;
- storage and archiving of the sonogram;
- possible transmission of the sonogram to a remote central server.

8. Kit for auscultation of the sound signals emitted by the organs of the human or animal body for diagnostic purposes comprising a stethoscope (10) according to one or more of the preceding claims 1 to 6 and a software application for the digital acquisition, processing and management of the signals detected by means of the stethoscope.
